# EUROPEAN PATENT APPLICATION

(11) **EP 4 527 915 A1**
(43) Date of publication of application: **26.03.2025**
(21) Application number: 24199579.4
(22) Date of filing: 10.09.2024
(51) Int. Cl.: C12M 1/107, B01D 5/00, C12M 1/00, F17D 1/02, F17D 3/14

(54) **SYSTEM FOR DRAINING CONDENSATE IN BIOGAS PIPELINES**

(30) Priority: 19.09.2023 IT 202300019266
(71) Applicant: Corradi & Ghisolfi Srl, 26010 Corte de' Frati (CR) (IT)
(72) Inventor: CORRADI, Bruno, 26010 Corte De Frati (CR) (IT); CORRADI, Paolo, 26010 Corte De Frati (CR) (IT)
(74) Representative: Fezzardi, Antonio

(57) **Abstract**

A system for draining condensate in biogas pipelines comprises a main pipeline (1) for biogas and a secondary pipeline (2) for collecting and draining condensate, which secondary pipeline (2) is located at a lower level than the main pipeline and communicates with it by means of appropriate check valves (3).

Said primary (1) and secondary (2) pipelines connect the biogas production plant (IP) to the collection section for utilization away from the plant itself, or to the section for upgrading (UPG) to biomethane for inputting it into the distribution network

## Description

The present invention relates to the field of biogas production plants, with particular reference to pipelines for transferring biogas produced in anaerobic digesters to storage sections or to the upgrading section for inputting it into the distribution network toward gas consumers.

More specifically, the invention relates to a system for draining condensate present in biogas pipelines.

Currently, the biogas produced in this manner is substantially used on site, usually within 150-200 meters of the production site, to fuel endothermic engines of electric power generators and recovering at least part of the heat associated with such endothermic engines by warming greenhouses, heating water, etc.

However, in many cases the biogas produced must be sent either to a collection section (where the upgrading takes place) to be able to input it into the distribution network, or to a section of utilization away from the production site.

It is known that the gas produced by the anaerobic matrix (named biogas) has a very high moisture content (substantially saturated), on the order of about 90%. This very high moisture content makes it practically impossible to transport through normal pipelines because that moisture condenses in a very short time, obstructing the pipeline and making biogas transfer practically impossible. By way of example, about 30 liters of water are present in 500 normal m³ of gas.

This means that if the pipeline is 100-200 meters long, with the right slopes, one can think of providing a siphon at mid-pipeline, which discharges condensate into a sump and is emptied with a bilge pump, but if the pipeline is longer (e.g., one or two kilometers), many sumps and many pumps must be provided with very high implementation and maintenance costs.

It is the main object of the present invention to overcome these problems by providing a condensate drainage system which can also be applied to long pipelines.

This was achieved by providing a system comprising a main biogas pipeline and a secondary condensate collection and drainage pipeline, located at a lower level than the main pipeline and communicating with it by means of appropriate check valves, in which said primary and secondary pipelines connect the biogas production plant to:
- the collection (upgrading) section to remove CO₂ and obtain biomethane, which can be input into the distribution network;
   or
- the biogas utilization section, e.g., such as a co-generator, boiler, etc.

To do this, the wet biogas which is input from the production plant side is pressurized into the main pipeline with a blower which has a head of a few mbar (e.g., 10-50 mbar), strictly necessary to overcome pressure drops, while on the utilization or upgrading side there is a compressor which - when necessary - empties the secondary pipeline of the condensate which has accumulated by temporarily pumping the biogas into the secondary pipeline with a pressure of 1.5-2 bar, so as to push said condensate back into the production plant itself.

Both pipelines are preferably buried, and the secondary one, which is placed at a level below the main one, is preferably placed on the side with respect to the main pipeline.

A better understanding of the invention will be achieved by means of the following description and with reference to the accompanying figures, which illustrate a preferred embodiment, by way of example.

In the drawings:
Figure 1 is a view diagrammatically showing the main components of the drainage system of the present invention;
Figures 2, 3 and 4, are respectively enlarged views of three details of the invention shown in Fig. 1;
Figure 5 is a side view of the main and secondary pipelines of the drainage system in Fig. 1;
Figure 6 is a plan view from above of the main and secondary pipelines, corresponding to Fig. 5;
Figures 7, 8 and 9, are respectively enlarged views of two details of the invention and of a section taken along A-A shown in Fig. 5.

With reference to the figures described above, the drainage system of present invention substantially comprises:
- a main biogas pipeline (1), which connects the biogas production plant (IP) to the biogas upgrading (UPG)/ utilization sections;
- a secondary condensate pipeline (2), which connects the biogas upgrading (UPG)/ utilization sections to the biogas production plant (IP) and which is arranged at a constantly lower level than the main pipeline (1);
- a plurality of check valves (3) disposed between said main and secondary pipelines (1, 2), configured to connect the corresponding lower part of the main pipeline (1) to the secondary pipeline (2), so that the liquid condensate falls by gravity from the main pipeline for the transit of biogas to the secondary condensate collection pipeline;
- at least one blower (SB) connected to a starting end of main pipeline (1) to send the biogas therein and having the head necessary to reach the biogas upgrading (UPG)/ utilization sections;
- at least one compressor (C) connected to the end of the main (1) and secondary (2) pipelines to pump, when necessary, a small portion of the biogas from the main pipeline (1) towards the secondary pipeline (2), with the pressure needed to push the condensate backwards towards the biogas production plant (IP).

According to a first particular feature of the invention, each of said check valves (3) is equipped with a ball made of polyethylene or other rigid material capable of cooperating with an appropriate circular seat to temporarily close the same valve and prevent the liquid condensate from passing from the secondary pipeline (2) to the main pipeline (1).

In the described preferred embodiment, by way of non-limiting example, said ball is preferably filled with nylon and lined with rubber, and the valve (3) is configured so that the ball is normally lowered, leaving the condensate drainage free, and is raised to occlude the upper circular seat by effect of Archimedes' principle, only when the level of condensate within the secondary pipe (2) rises due to its filling and/or due to the pressure generated by the compressor (C).

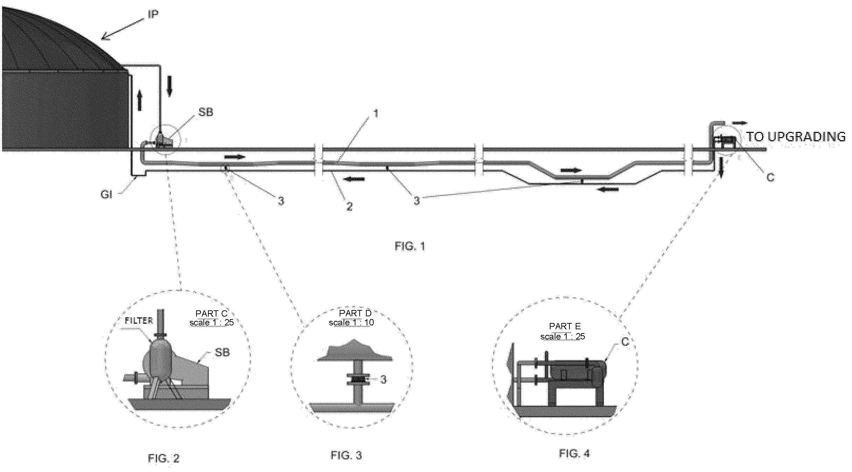

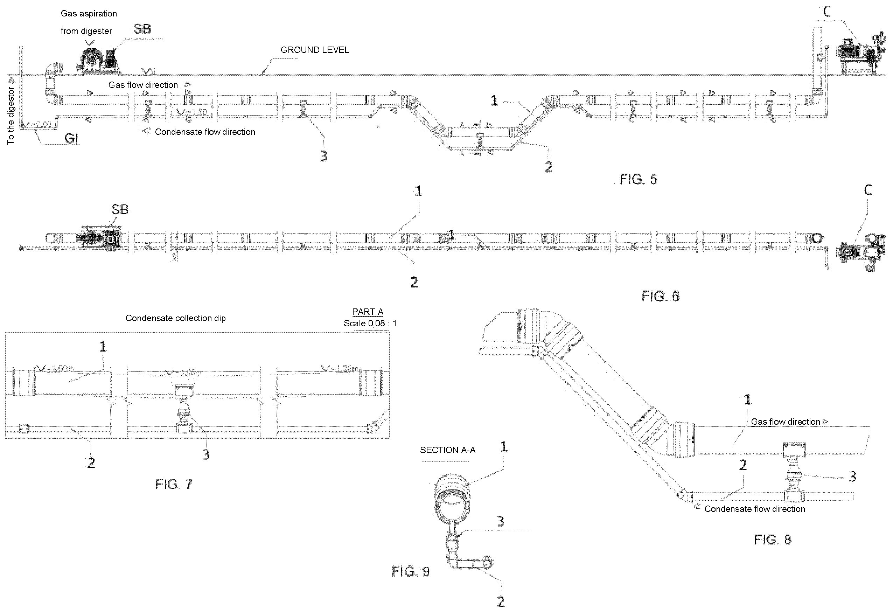

A second particular feature of the invention relates to the fact that there is a "hydraulic seal" (GI) at the final segment of the secondary condensate pipeline (2), in which the secondary pipeline has (Figs. 1 and 5):
- a segment that suddenly drops downwards, preferably for at least 50 cm;
- a substantially horizontal segment;
- a segment that rises upwards until it reaches the entrance of the biogas production plant from above.

Advantageously, the presence of such hydraulic seal prevents the biogas, sent by compressor (C) into the secondary pipeline (2) for pushing the liquid condensate into the biogas production plant, from accidentally emptying the entire secondary pipeline and causing a sudden pressure drop. During the operation of the compressor (C), it is preferable to increase the head of the blower (SB) to keep the value of biogas flow rate towards upgrading/utilization substantially constant.

A third particular feature of the invention consists in that each segment of the main pipeline (1) located in the close vicinity of each check valve (3) is at a slightly lower level than the adjacent pipeline segments, e.g., with a lower level of about 5 cm, to facilitate the collection of the condensate (Fig. 7).

Finally, if the two pipelines must pass under a ditch/road/cable duct, said main and secondary pipelines are provided with special shaped zones consisting of two sloping side segments and one horizontal center segment (Fig. 8). In these cases, a check valve (3) is provided in the horizontal central segment because the presence of a segment lower than the rest of the pipeline (1) is a natural condensate accumulation section.

In the preferred embodiment shown in the drawings, by way of non-limiting example, the main pipeline (1) has an internal diameter of 200-250 mm and the ball of each check valve has a diameter of about 60 mm.

The pipelines are preferably made of polyethylene, and the valves (3) are installed at a distance from each other of about 25 m, in addition to those at (main and secondary) pipeline segments that pass under ditches/roads/cable ducts, if any.

## Claims

1. A system for draining condensate in biogas pipelines, **characterized in that** it comprises a main biogas pipeline (1) and a secondary condensate pipeline (2) for collecting and draining the condensate, the secondary pipeline (2) being located at a lower level than the main pipeline and communicating therewith by means of appropriate check valves (3), wherein said primary pipeline (1) and secondary pipeline (2) connect the biogas production plant (IP) to the collection section for the utilization of biogas away from the plant itself or to the section for upgrading (UPG) to biomethane for inputting it into the distribution network.

2. A system according to claim 1, **characterized in that** it substantially comprises:
- a main biogas pipeline (1) that connects the biogas production plant (IP) to the upgrading (UPG)/ utilization sections;
- a secondary condensate pipeline (2) that connects the upgrading (UPG)/ utilization sections to the biogas production plant (IP) and is arranged at a constantly lower level than the main pipeline (1);
- a plurality of check valves (3), disposed between said main and secondary pipelines (1, 2) and configured to connect a corresponding lower part of the main pipeline (1) to the secondary pipeline (2), so that the liquid condensate falls by gravity from the main pipeline (1) for the transit of biogas to the secondary pipeline (2) for the collection of condensate;
- at least one blower (SB) connected to a starting end of main pipeline (1) to send the biogas therein, with the head necessary to reach the biogas upgrading (UPG) section;
- at least one compressor (C) connected to the end of the main (1) and secondary (2) pipelines to pump, when necessary, the biogas from the main pipeline to the secondary pipeline, with the pressure needed to push the condensate present in the secondary pipeline (2) backwards towards the biogas production plant.

3. A system according to the preceding claim, **characterized in that** each of said check valves (3) is equipped with a ball capable of cooperating with an appropriate circular seat to temporarily close the valve and prevent the liquid condensate from passing upwards from the secondary pipeline (2) to the main pipeline (1), when the compressor (C) is in operation.

4. A system according to one or more of the preceding claims, **characterized in that** a "hydraulic seal" (GI) is provided at the final segment of secondary condensate pipeline (2) on the side of biogas production plant (IP), wherein the secondary pipeline comprises:
- a segment that suddenly drops downwards, preferably for at least 50 cm;
- a substantially horizontal segment;
- a segment that rises upwards until it reaches the entrance of the biogas production plant from above; said hydraulic seal being capable to prevent the biogas, sent by compressor (C) into the secondary pipeline (2) for pushing the liquid condensate that occupied it to the biogas production plant, from accidentally emptying the entire secondary pipeline, thus causing a sudden pressure drop.

5. A system according to one or more of the preceding claims, **characterized in that** during the operation of the compressor (C), the head of the blower (SB) is increased to a value sufficient to keep the biogas flow rate towards the upgrading section (UPG) unchanged.

6. A system according to one or more of the preceding claims, **characterized in that** each segment of the main pipeline (1), which is located in the close vicinity of each check valve (3), is at a slightly lower level than the adjacent pipeline segments, for example a lower level of about 5 cm, to facilitate the collection of the condensate.

7. A system according to one or more of the preceding claims, **characterized in that** if the two pipelines must pass under a ditch/road/cable duct, said main (1) and secondary (2) pipelines are equipped with appropriately shaped zones consisting of two inclined lateral segments and a horizontal central segment, wherein a check valve (3) is always provided in the horizontal central segment, since the presence of a segment lower than the rest of the pipeline (1) provides a natural accumulation section for the condensate.

8. A system according to one or more of the preceding claims, **characterized in that** the main pipeline (1) has an internal diameter of 200-250 mm and the ball of each check valve (3) has a diameter of about 60 mm.

9. A system according to one or more of the preceding claims, **characterized in that** said pipelines (1, 2) are made of polyethylene and/or the check valves (3) are installed at a mutual distance of about 25 m.

10. A system according to one or more of the preceding claims, **characterized in that** the wet biogas input from the biogas production plant (IP) side is pressurized in the main pipeline (1) by a blower (SB) that has a head of at least 10-50 mbar, while a compressor (C) is provided on the upgrading section (UPG) side, which, when the secondary pipeline must be emptied from the accumulated condensate, it temporarily pumps the biogas into the secondary pipeline (2) with a pressure of 1.5-2 bar in order to push said condensate inside the same biogas production plant.

11. A system according to one or more of the preceding claims, **characterized in that** both the pipelines (1, 2) are buried and/or the secondary pipeline (2) is placed on the side with respect to the main pipeline (1).
